# EUROPÄISCHE PATENTANMELDUNG

(11) **EP 0 592 876 A1**
(43) Veröffentlichungstag der Anmeldung: **20.04.1994**
(21) Anmeldenummer: 93115784.6
(22) Anmeldetag: 30.09.1993
(51) Int. Cl.: A61K 7/48, A61K 7/06, A61K 31/045, A61K 31/23, A61K 31/08, A61K 31/77, A61K 31/765, A23L 3/3517, A23L 3/3481

(54) **Antimycotische kosmetische und dermatologische Zubereitungen**

(30) Priorität: 10.10.1992 DE 4234188
(71) Anmelder: Beiersdorf Aktiengesellschaft, D-20245 Hamburg (DE)
(72) Erfinder: Wolf, Florian, Dr., D-20251 Hamburg (DE); Kahlert, Michael, D-22041 Hamburg (DE)

(57) **Zusammenfassung**

Die Verwendung ethoxylierter und/oder propoxylierter organischer Verbindungen als antimycotisches Wirkprinzip in kosmetischen oder dermatologischen Zubereitungen.

## Beschreibung

Die vorliegende Erfindung betrifft antimycotisch wirksame und gegen den Befall mit Mycobionten geschützte Zubereitungen, bevorzugt kosmetischen oder dermatologischen Zubereitungen.

Pilze, auch Fungi [fungus = lat. Pilz], Mycota [mykes = grch. Pilz] oder Mycobionten genannt, zählen zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Schließlich können durch Mycobiontenbefall Zubereitungen, insbesondere Nahrungsmittel, aber auch Kosmetika und dergleichen zerstört werden. Der Schutz vor Infektion erstreckt sich daher auch auf die Produkte selbst.

Behandelt werden Dermatomycosen medikamentös oder mit anderen Methoden, beispielsweise mit Lichtbestrahlung. Gängige Medikamente enthalten Salicylsäure, Kresol, 1-Menthol und andere, die alle äußerlich angewandt werden und regelmäßig gute Heilerfolge zeitigen.

Dennoch haben die bisher bekannten antimycotisch wirksamen Mittel den Nachteil daß sie unangenehm riechen und/oder die beeinträchtigte Hautpartie zusätzlich reizen. In schweren Fällen von Dermatomycosen kann durch das Medikament sogar Schmerz ausgelöst werden.

Die Aufgabe der vorliegenden Erfindung war also antimycotisch wirksame Mittel zur Verfügung zu stellen, welche die Nachteile des Standes der Technik nicht aufweisen. Insbesondere sollten Wirkstoffe zugängig gemacht werden, die folgende Bedingungen erfüllen:
1) Die biologischen Vorgänge der Haut dürfen nicht beeinträchtigt werden.
2) Die Wirkstoffe sollen keinen ausgeprägten Eigengeruch besitzen.
3) Sie sollen bei Überdosierung oder sonstiger nicht bestimmungsgemäßer Anwendung unschädlich sein.
4) Sie sollen sich nach wiederholter Anwendung nicht auf der Haut anreichern.
5) Pathogene und fakultativ pathogene Mycobionten sollen wirksam bekämpft werden, die physiologische Mikroflora der Haut im übrigen aber geschont werden.
6) Die wirksamen Prinzipien sollen sich gut in übliche kosmetische oder dermatologische Formulierungen einarbeiten lassen.
7) Die Wirkstoffe sollen die Haut nicht reizen.
8) Zubereitungen, diese Wirkstoffe enthaltend, sollen ebenfalls vor Befall mit Mycobionten geschützt sein.

Es wurde gefunden, und darin liegt die Lösung all dieser Aufgaben, daß
die Verwendung ethoxylierter und/oder propoxylierter organischer Verbindungen als antimycotisches Wirkprinzip in kosmetischen oder dermatologischen Zubereitungen
den Nachteilen des Standes der Technik abhilft.

Unter Ethoxylierung wird der chemische Prozeß verstanden, bei welchem eine odere mehrere Ethoxygruppen an eine chemische Substanz geknüpft werden.

Die Ethoxygruppe ist durch die Struktur [-O-CH₂-CH₂-] gekennzeichnet. Als Ethoxylierungagens wird üblicherweise Ethylenoxid (Synonyme: Oxiran, Oxa-Cyclopropan) benutzt.

Ethylenoxid reagiert mit bestimmten reaktiven Gruppen, beispielsweise
a) Hydroxylgruppen: 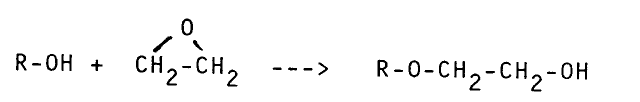 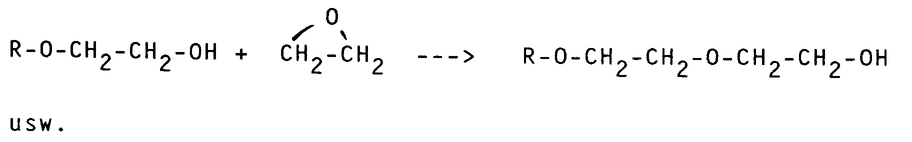
b) Säuregruppen

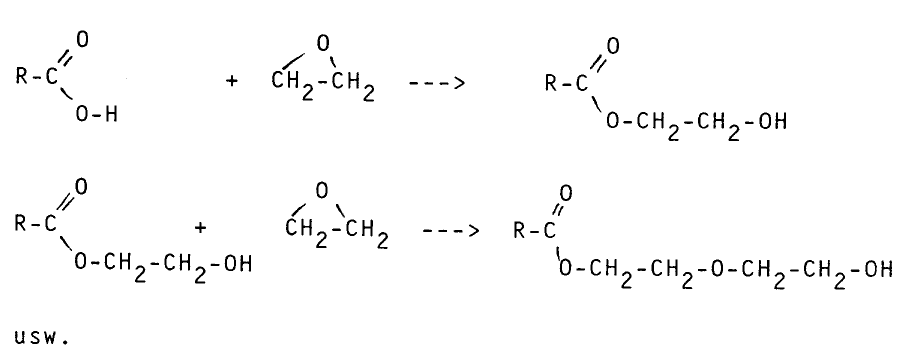

Unter Propoxylierung wird in Analogie zur Ethoxylierung der chemische Prozeß verstanden, bei welchem eine odere mehrere Propoxyruppen an eine chemische Substanz geknüpft werden.

Die Propoxygruppe ist durch die Strukturen 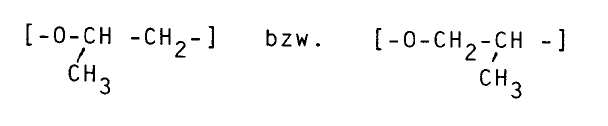
gekennzeichnet. Als Propoxylierungsagens wird üblicherweise Propylenoxid (Synonym: Methyloxiran) benutzt.

Propylenoxid reagiert in Analogie zu Ethylenoxid mit bestimmten reaktiven Gruppen, beispielsweise
a) Hydroxylgruppen: 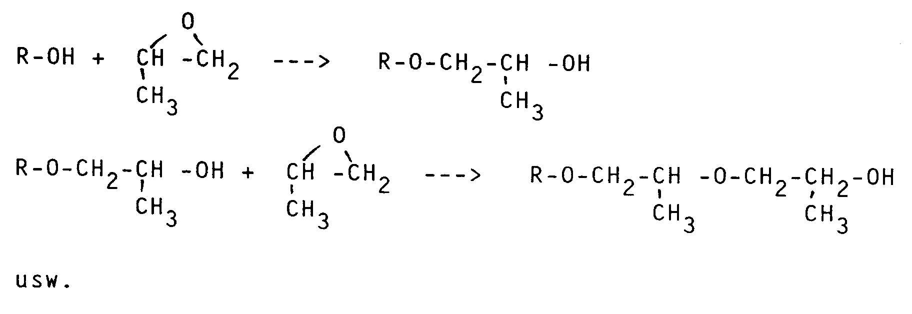
b) Säuregruppen

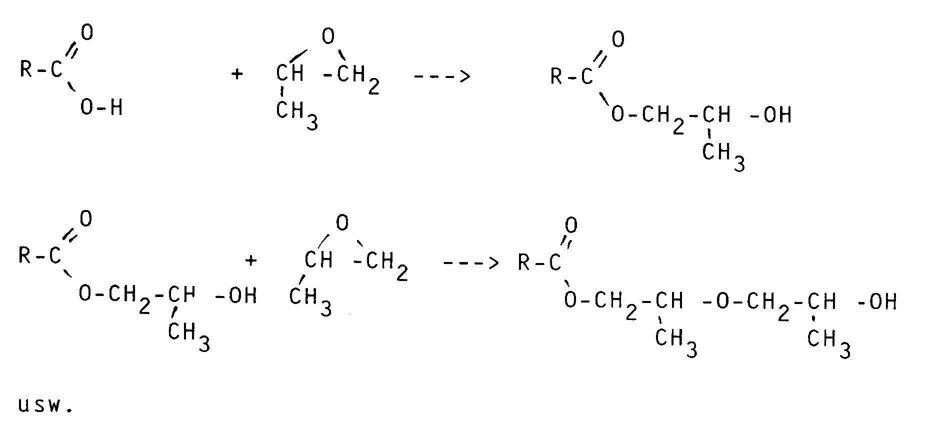

Entsprechend den voranstehend aufgezeigten Reaktionsschemata werden die bevorzugten ethoxylierten und/oder propoxylierten organischen Verbindungen gewählt aus der Gruppe der einfach oder mehrfach ethoxylierten und/oder propoxylierten Alkohole und Fettsäuren, wobei diese Alkohole und Fettsäuren unabhängig voneinander eine oder mehrere Alkohol- bzw. Säurefunktion aufweisen oder auch eine oder mehrere Alkohol- und eine oder mehrere Säurefunktionen in einem Molekül vereinen können. Sie können gesättigt oder ungesättigt, verzweigt oder unverzweigt sein. Bevorzugt werden sie aus der Gruppe der Alkohole bzw. Fettsäuren mit 5 - 25 Kohlenstoffatomen gewählt.

Ferner ist gegebenenfalls von Vorteil, wenn die bei der Ethoxylierung bzw. Propoxylierung gebildete terminale Hydroxylgruppe ebenfalls verethert bzw. verestert vorliegt.

Besonders bevorzugte ethoxylierte organische Verbindungen werden gewählt aus der Gruppe
R'-(O-CH₂-CH₂)ₙ-O-R
Dabei wird R gewählt aus der Gruppe
H-, C₅₋₂₅-Alkyl-, 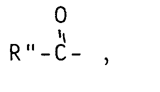
R' wird gewählt aus der Gruppe
C₅₋₂₅-Alkyl-, 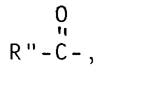
R'' wird gewählt aus der Gruppe C₅₋₂₅-Alkyl-.
n stellt eine Zahl von 1 bis 50 dar.

Besonders bevorzugte propoxylierte organische Verbindungen werden gewählt aus der Gruppe 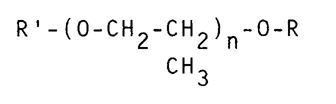
Dabei wird R gewählt aus der Gruppe
H-, C₅₋₂₅-Alkyl-, 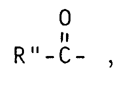
R' wird gewählt aus der Gruppe
C₅₋₂₅-Alkyl-, 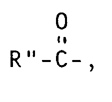
R'' wird gewählt aus der Gruppe C₅₋₂₅-Alkyl-.
n stellt eine Zahl von 1 bis 50 dar.

Bevorzugte ethoxylierte bzw. propoxylierte organische Verbindungen im Sinne der vorliegenden Erfindung sind insbesondere Verbindungen, bei welchen R aus der Gruppe H, C₁₂₋₂₀-Alkyl, wobei C₁₈ besonders bevorzugt wird, R' aus der Gruppe C₁₂₋₂₀-Alkyl, wobei C₁₈ besonders bevorzugt wird, gewählt werden und n eine Zahl von 5 - 30 darstellt.

Ganz besonders bevorzugt sind solche ethoxylierte organische Verbindungen, welche aus der Gruppe der nach der CTFA-Nomenklatur Ceteth-X, Ceteareth-X, Steareth-X genannt werden, wobei X eine Zahl von 1 - 50 darstellen kann, bevorzugt von 5 - 30.

Es hat sich in erstaunlicher Weise herausgestellt, daß die erfindungsgemäßen ethoxylierten bzw. propoxylierten organischen Verbindungen das Wachstum von Mycobionten verhindern.

Insbesondere sind die erfindungsgemäßen ethoxylierten bzw. propoxylierten organischen Verbindungen befähigt, daß Wachstum von Hefen, insbesondere der Pityrosporum-Arten, namentlich Pityrosporum ovale, zu verhindern.

Es hat sich ferner herausgestellt, daß die erfindungsgemäßen ethoxylierten bzw. propoxylierten organischen Verbindungen die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß ist somit auch ein Verfahren zur Bekämpfung seborrhoischer Erscheinungen, insbesondere Kopfschuppen, sowie die Prophylaxe gegen seborrhoische Erscheinungen, insbesondere Kopfschuppen.

Schließlich hat sich herausgestellt, daß die erfindungsgemäßen ethoxylierten bzw. propoxylierten organischen Verbindungen den Verderb organischer Substanz, insbesondere kosmetischer und dermatologischer Zubereitungen, durch den Befall mit Mycobionten verhindern können, wenn sie diesen Zubereitungen zugesetzt werden.

Erfindungsgemäß sind somit auch ein Verfahren zur Bekämpfung von Mycobionten, dadurch gekennzeichnet, daß ethoxylierte und/oder propoxylierte organische Verbindungen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten ethoxylierte und/oder propoxylierte organische Verbindungen in wirksamer Menge zugegeben werden.

Erfindungsgemäß werden die ethoxylierten bzw. propoxylierten organischen Verbindungen bevorzugt in kosmetischen oder dermatologischen Zusammensetzungen eingesetzt einem Gehalt von 0,01 - 50,0 Gew.-% , insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäßen ethoxylierten bzw. propoxylierten organischen Verbindungen, ganz besonders vorteilhaft 0,5 - 3,0 Gew.-%, jeweils bezogen auf das Gesamtgewicht der Zusammensetzung.

Die erfindungsgemäßen ethoxylierten bzw. propoxylierten organischen Verbindungen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Ganz besonders vorteilhaft liegen die erfindungsgemäßen ethoxylierten bzw. propoxylierten Verbindungen in Form von Antischuppen-Shampoos vor.

Es ist auch möglich und gegebenenfalls vorteilhaft, die erfindungsgemäßen ethoxylierten bzw. propoxylierten organischen Verbindungen mit anderen Wirkstoffen zu kombinieren, beispielsweise mit antimikrobiell wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,0 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine erfindungsgemäße ethoxylierte oder propoxylierte organische Verbindung im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens eine erfindungsgemäße ethoxylierte oder propoxylierte organische Verbindung. Die Menge der verwendeten Polymeren liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die mindestens eine erfindungsgemäße ethoxylierte oder propoxylierte organische Verbindung enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben mindestens eine erfindungsgemäße ethoxylierte oder propoxylierte organische Verbindung und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen ethoxylierten bzw. propoxylierten organischen Verbindungen in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Anhand der folgenden Versuche soll die erstaunliche antimykotische Wirkung der erfindungsgemäßen ethoxylierten bzw. propoxylierten organischen Verbindungen demonstriert werden:

### Versuch 1

### Testlösungen:

- TY-Medium: 10 g Trypton + 5 g Hefeextrakt + 10 g NaCl, aufgelöst in 1000 ml H₂O, gepuffert auf pH 7,4 , autoklaviert.
- Testverbindung 1:: Polyoxyethylen-20-stearylether
- Testverbindung 2:: Polyoxyethylen-20-isostearylalkohol
- Testverbindung 3:: Polyoxyethylen-21-stearylalkohl
- Testverbindung 4:: Polyoxyethylen-5-cetyl/stearylether
- Testverbindung 5:: Polyoxyethylen-25-Fettalkohol
- Testverbindung 6:: Polyoxyethylen-6-cetyl/stearylether
- Testverbindung 7:: Polyoxyethylen-C₁₂-C₁₅-Alkylether

Pityrosporum ovale, isoliert von drei Patienten mit Pityrosporum-Follikulitis, Pityriasis versicolor oder seborrhoischem Ekzem wurde in einer Mischung aus 50 % TY-Medium, 30 % Olivenöl und 20 % Tween 80 suspendiert. Die drei verschiedenen Hefestämme wurden in drei parallelen Ansätzen auf einer Selektivagarplatte für pathogene Pilze (Merck AG, Darmstadt) gleichmäßig ausgespatelt. Mit Hilfe steriler Korkbohrer wurden in jede Platte mehrere kreisrunde Vertiefungen gestanzt, In die Vertiefungen mit einem Durchmesser von 5 mm wurden jeweils 25 µl der Testlösungen gegeben. Die Testlösungen stellten 1%-ige und 10%-ige Lösungen der Testsubstanzen in Ethanol dar.

Nach dreistündiger Inkubation bei 37° C wurde das verdunstete Lösungsmittel durch je 25 µl Jojobaöl ersetzt und die Platten anschließend unter Beibehaltung der Wachstumsbedingungen drei Tage inkubiert. Während dieser Zeit diffundierten die Testsubstanzen in den die Vertiefungen umgebenden Nährboden.

Antimycotisch wirksame Testverbindungen zeigten nach dreitägiger Inkubation einen mehr oder weniger deutlichen konzentrischen Hemmhof um die Vertiefungen. Die Größe dieser Hemmhöfe, wiedergegeben in Tabelle 1, ist bei
vergleichbarer Substanzkonzentration ein Maß für die antimycotische Wirksamkeit der Testverbindungen.

**Tabelle 1**

| | mittlerer Hemmhof-Durchmesser [mm] | |
|---|---|---|
| | 1%-ige Lösung | 10%-ige Lösung |
| Testlösung 1 | 10,0 | 15,5 |
| Testlösung 2 | 11,0 | 12,0 |
| Testlösung 3 | 7,0 | 11,0 |
| Testlösung 4 | 8,5 | 9,5 |
| Testlösung 5 | 7,0 | 13,0 |
| Testlösung 6 | 9,5 | 14,0 |
| Testlösung 7 | 9,5 | 15,0 |

### Versuch 2

- Testverbindung 8 :: Polyoxyethylen-??-Dodecylether
- Testverbindung 9 :: Polyoxyethylen-20-Stearylether
- Testverbindung 10:: Polyoxyethylen-20-isostearylalkohol
- Testverbindung 11:: Polyoxyethylen-21-stearylalkohol
- Testverbindung 12:: Polyoxyethylen-5-Cetyl/stearylether
- Testverbindung 13:: Polyoxyethylen-25-Fettalkohol
- Testverbindung 14:: Polyoxyethylen-6-Cetyl/Stearylether + Stearylalkohol
- Testverbindung 15:: Polyoxyethylen-C₁₂-C₁₅-Alkylether

Von den Testverbindungen 8 - 15 wurden ethanolische Lösungen der Konzentrationen 0,5 %, 1,0 %, 5,0 % und 10,0 % hergestellt und in den Hemmhof-Test eingeführt. Die Versuchsdurchführung entspricht in ihrem Ablaufe dem zu Versuch 1 geschilderten. Die Größe der betreffenden Hemmhöfe ist in Tabelle 2 wiedergegeben.

**Tabelle 2**

| | mittlerer Hemmhof-Durchmesser [mm] | | | | |
|---|---|---|---|---|---|
| | Konz.: | 0,5 % | 1,0 % | 5,0 % | 10,0 % |
| Testlösung | 8 | 14,0 | 15,5 | 18,0 | 20,7 |
| Testlösung | 9 | 10,7 | 14,0 | 18,7 | 20,7 |
| Testlösung | 10 | 9,0 | 9,0 | 15,0 | 17,3 |
| Testlösung | 11 | 10,0 | 11,7 | 12,0 | 12,7 |
| Testlösung | 12 | 11,0 | 14,0 | 16,5 | 16,5 |
| Testlösung | 13 | 10,0 | 11,5 | 15,7 | 18,3 |
| Testlösung | 14 | 10,5 | 11,0 | 13,7 | 14,0 |
| Testlösung | 15 | 9,0 | 12,7 | 17,0 | 20,0 |

Die nachfolgenden Beispiele sollen die vorliegende Erfindung verdeutlichen, ohne sie einzuschränken.

### Beispiel 1

| Feuchtigkeitslotion | |
|---|---|
| | Gew.-% |
| Ceteareth-6 + Stearylalkohol ("Cremophor A 6) | 2,00 |
| Ceteareth-25 ("Cremophor A 25") | 2,00 |
| Cetearyloctanoat ("Luvitol EHO") | 6,00 |
| Cetylstearylalcohol | 0,50 |
| Glycerinmonostearat | 6,00 |
| Paraffinöl DAB 9 | 6,00 |
| Tocopherylacetat | 3,00 |
| 1,2-Propylenglycol | 3,00 |
| Konservierungsmittel, Parfum, Stabilisatoren usw. | nach Belieben |
| Wasser | ad 100,00 |

### Beispiel 2

| Reinigungslotion | |
|---|---|
| | Gew.-% |
| Ceteareth-6 + Stearylalkohol ("Cremophor A 6") | 1,50 |
| Ceteareth-25 ("Cremophor A 25") | 1,50 |
| Cetearyloctanoat ("Luvitol EHO") | 4,00 |
| Glycerinmonostearat | 2,50 |
| Cetylalkohol | 1,50 |
| Paraffinöl DAB 9 | 4,00 |
| 1,2-Propylenglycol | 3,00 |
| Konservierungsmittel, Parfum, Stabilisatoren usw. | nach Belieben |
| Wasser | ad 100,00 |

### Beispiel 3

| Hautschutzcrème | |
|---|---|
| | Gew.-% |
| Ceteareth-6 + Stearylalkohol ("Cremophor A 6") | 2,00 |
| Ceteareth-25 ("Cremophor A 25") | 2,00 |
| Glycerinmonostearat | 5,00 |
| Cetylalkohol | 5,00 |
| Paraffinöl DAB 9 | 9,00 |
| Dimethicone ("Abil 100") | 1,00 |
| Polyquaternium-16 ("Luviquat FC 370") | 5,00 |
| Glycerin | 10,00 |
| Konservierungsmittel, Parfum, Stabilisatoren usw. | nach Belieben |
| Wasser | ad 100,00 |

### Beispiel 4

| Badeöl, wasserlöslich | |
|---|---|
| | Gew.-% |
| PEG-12-C₁₂-C₁₅-alkylether ("Mulsifan RT 203/80") | 8,00 |
| Polysorbat 20 ("Mulsifan RT 141") | 30,00 |
| Linoleamid DEA ("Purton SFD") | 3,00 |
| Extrakt aus Rosmarinblättern, dickflüssig, wasserlöslich | 5,00 |
| Rosmarinöl | 3,00 |
| Konservierungsmittel, Parfum, Stabilisatoren usw. | nach Belieben |
| Wasser | ad 100,00 |

### Beispiel 5

| Tagescrème, schwach fettend | |
|---|---|
| | Gew.-% |
| Triceteareth-4 Phosphat ("Hostacerin KW 340 N") | 10,00 |
| Ceteareth-3 ("Hostacerin T-3") | 3,00 |
| Cetylalkohol DAB 7 | 7,00 |
| Isopropylmyristat | 7,00 |
| Paraffinöl DAB 7, dickflüssig | 1,80 |
| GS-Salbenwachs (G.Schütz) | 2,00 |
| Konservierungsmittel, Parfum, Stabilisatoren usw. | nach Belieben |
| Wasser | ad 100,00 |

### Beispiel 6

| Vanishing-Crème | |
|---|---|
| | Gew.-% |
| Steareth-20 ("Genapol S-200") | 3,00 |
| Ceteareth-3 ("Hostacerin T-3") | 5,00 |
| Stearinsäure | 10,00 |
| Paraffinöl DAB 7, dickflüssig | 15,00 |
| Konservierungsmittel, Parfum, Stabilisatoren usw. | nach Belieben |
| Wasser | ad 100,00 |

### Beispiel 7

| Antischuppenshampoo klar | |
|---|---|
| | Gew.-% |
| Allantoin | 0,20 |
| Natrium-alkyl-polyglycolethersulfat ("Genapol LRO flüssig") | 25,00 |
| Natrium-alkyl-polyglycolethersulfat ("Genapol AMG") | 10,00 |
| Polyoxyethylen-25-Fettalkohol | 10,00 |
| NaCl | 1,10 |
| Konservierungsmittel, Parfum, Stabilisatoren usw. | nach Belieben |
| Wasser | ad 100,00 |

### Beispiel 8

| Antischuppenshampoo mit Perlglanz | |
|---|---|
| | Gew.-% |
| Natrium-alkyl-polyglycolethersulfat ("Genapol LRO flüssig") | 40,00 |
| Natrium-alkyl-polyglycolethersulfat ("Genapol PGL") | 4,00 |
| Fettsäure-alkylolamidpolyglycolether ("Genagen CAB") | 10,00 |
| Polyoxyethylen-25-Fettalkohol | 10,00 |
| NaCl | 0,50 |
| Konservierungsmittel, Parfum, Stabilisatoren usw. | nach Belieben |
| Wasser | ad 100,00 |

Die erfindungsgemäßen Formulierungen gemäß den Beispielen 1 - 8 sind hervorragend gegen Befall von Mycobionten geschützt. Die Antischuppenshampoos gemäß den Beispielen 7 und 8 zeichnen sich durch ausgezeichnete Antischuppenwirkung aus.

## Patentansprüche

1. Die Verwendung ethoxylierter und/oder propoxylierter organischer Verbindungen als antimycotisches Wirkprinzip in kosmetischen oder dermatologischen Zubereitungen.

2. Verwendung nach Anspruch 1, dadurch gekennzeichnet, daß die ethoxylierten und/oder propoxylierten organischen Verbindungen gewählt aus der Gruppe der einfach oder mehrfach ethoxylierten und/oder propoxylierten Alkohole und Fettsäuren, wobei diese Alkohole und Fettsäuren unabhängig voneinander eine oder mehrere Alkohol- bzw. Säurefunktion aufweisen oder auch eine oder mehrere Alkohol- und eine oder mehrere Säurefunktionen in einem Molekül vereinen können, wobei die Alkohole und/oder Fettsäuren gesättigt oder ungesättigt, verzweigt oder unverzweigt sein können und bevorzugt aus der Gruppe der Alkohole bzw. Fettsäuren mit 5 - 25 Kohlenstoffatomen gewählt werden.

3. Verwendung nach Anspruch 2, dadurch gekennzeichnet, daß die bei der Ethoxylierung bzw. Propoxylierung gebildete terminale Hydroxylgruppe ebenfalls verethert bzw. verestert vorliegt.

4. Verwendung nach Anspruch 1, dadurch gekennzeichnet, daß die ethoxylierten organischen Verbindungen werden gewählt aus der Gruppe
R'-(O-CH₂-CH₂)ₙ-O-R ,
wobei R gewählt wird aus der Gruppe
H-, C₅₋₂₅-Alkyl-, 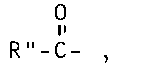 R' gewählt wird aus der Gruppe
C₅₋₂₅-Alkyl-, 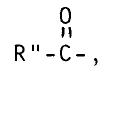 R'' gewählt wird aus der Gruppe C₅₋₂₅-Alkyl- ,
n eine Zahl von 1 bis 50 darstellt.

5. Verwendung nach Anspruch 1, dadurch gekennzeichnet, daß die propoxylierten organischen Verbindungen werden gewählt aus der Gruppe 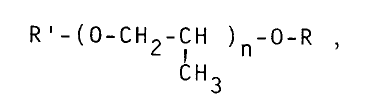 wobei R gewählt wird aus der Gruppe
H-, C₅₋₂₅-Alkyl-, 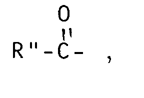 R' gewählt wird aus der Gruppe
C₅₋₂₅-Alkyl-, 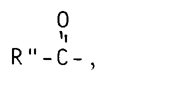 R'' gewählt wird aus der Gruppe C₅₋₂₅-Alkyl- ,
n eine Zahl von 1 bis 50 darstellt.

6. Verwendung nach Anspruch 4 oder 5, dadurch gekennzeichnet, daß R aus der Gruppe H, C₁₂₋₂₀-Alkyl, wobei C₁₈ besonders bevorzugt wird, R' aus der Gruppe C₁₂₋₂₀-Alkyl, wobei C₁₈ besonders bevorzugt wird, gewählt werden und n eine Zahl von 5 - 30 darstellt.

7. Verwendung nach Anspruch 1, dadurch gekennzeichnet, daß die ethoxylierten bzw. propoxylierten organischen Verbindungen in kosmetischen oder dermatologischen Zusammensetzungen in einer Konzentration von 0,01 - 50,0 Gew.-% , vorteilhaft 0,01 - 20,0 Gew.-%, insbesondere 0,01 - 10,0 Gew.-%, besonders bevorzugt 0,02 - 5,0 Gew.-%, ganz besonders vorteilhaft 0,5 - 3,0 Gew.-%, enthalten sind, jeweils bezogen auf das Gesamtgewicht der Zusammensetzung.

8. Verfahren zur Bekämpfung von Mycobionten, dadurch gekennzeichnet, daß ethoxylierte und/oder propoxylierte organische Verbindungen, gegebenenfalls in einem geeigneten kosmetischen oder dermatologischen Träger, mit dem durch Mycobionten kontaminierten Bereich in Kontakt gebracht werden.

9. Verfahren zum Schutze organischer Produkte vor dem Befall mit Mycobionten, dadurch gekennzeichnet, daß diesen organischen Produkten ethoxylierte und/oder propoxylierte organische Verbindungen in wirksamer Menge zugegeben werden.

10. Verfahren zur Bekämpfung seborrhoisches Erscheinungen, insbesondere Kopfschuppen, sowie die Prophylaxe gegen seborrhoische Erscheinungen, insbesondere Kopfschuppen.
